# EUROPEAN PATENT APPLICATION

(11) **EP 2 787 005 A1**
(43) Date of publication of application: **08.10.2014**
(21) Application number: 13161965.2
(22) Date of filing: 02.04.2013
(51) Int. Cl.: C07K 16/22

(54) **Targeted cancer immune therapy**

(71) Applicant: Activartis Biotech GmbH, 1090 Vienna (AT)
(72) Inventor: The designation of the inventor has not yet been filed
(74) Representative: Sonn & Partner Patentanwälte

(57) **Abstract**

The invention discloses the combination of an active cancer immune medicament and a passive immune-therapeutic drug, wherein the passive immune-therapeutic drug is a monoclonal antibody (MAB) specific for a tumour-associated antigen (TAA).

## Description

The present invention relates to cancer treatment medicaments and methods for treatment of cancer.

The immune system has two major functional parts for executing an immune response:
(i) The humoral arm is based on antibodies - IgG molecules - that via the IgG molecule's antibody-binding fragment Fab specifically binds to an antigen, forming antigen/antibody immune complexes comprised of several IgG and antigen molecules, thus immobilising and/or neutralising the antigen; the immune complexes bind via the IgG molecules' constant fragment (Fc) to IgG Fc-receptors on phagocytes, which triggers the phagocyte's activation and the release of pro-inflammatory cytokines; and the antigen/antibody complexes may activate the complement system.
(ii) The cellular arm is based on T-cells that either guide the immune system's activities (helper T-lymphocytes, HTL), or serve as effector cells that can kill pathogen-infected body cells (cytotoxic T-lymphocytes, CTL); antigen-presenting cells take up antigen molecules, break them down into peptide fragments, bind these peptides to major histocompatibility complex (MHC) molecules, and shuttle the MHC/peptide complexes to the antigen-presenting cell's membrane; when T-cells via their T-cell receptor bind to the MHC/peptide complexes, they are primed to detect and destroy body cells that present the same MHC/peptide molecules on their membrane.

Many million years of evolution selected for a two-armed immune defence mechanism. Thus it appears reasonable that strategies for cancer immune therapy should imitate this physiologic mechanism. However, cancer immune therapy in the past has either (i) relied on the immune system's humoral arm alone, using monoclonal antibodies (MAB) directed against tumour antigens, which is equivalent to a passive immune therapy; or (ii) it relied on an active vaccination approach aimed at triggering the immune system's cellular arm. Currently most advanced are cancer vaccine concepts based on tumour antigen-charged dendritic cells (DCs), the most potent immune stimulators among the class of antigen-presenting cells.

It is an objective of the present invention to provide improved means and methods for the treatment of cancer.

The present invention provides a combination of an active cancer immune medicament and a passive immune-therapeutic drug, wherein the passive immune-therapeutic drug is a monoclonal antibody (MAB) specific for a tumour-associated antigen (TAA).

The present invention relates to a Targeted Cancer Immune Therapy (TCIT) comprised of an active component (an active cancer immune medicament) and a passive component ("targeting component"; passive immune-therapeutic drug that is a MAB specific for a TAA). Forming of immune complexes comprised of the MAB medicament and TAAs in the tumour tissue causes release of pro-inflammatory cytokines from tumour-resident phagocytes via binding of such immune complexes to the phagocytes' IgG Fc-receptors that guide T cells primed against TAAs by the active cancer immune medicament into the tumour tissue. The TCIT of the present invention takes advantage of both arms of the immune system, the cellular and the humoral arm, combining them into a novel treatment paradigm for cancer and demonstrating its superior effectiveness.

The active cancer immune medicament according to the present invention can be regarded as the component that provides the "active cancer immune therapy" (wherein the active cancer immune medicament is administered to a patient in an effective amount). Active cancer immune therapy (by administration of the active cancer immune medicament) in the context of this invention means that effector mechanisms are triggered once the cancer immune medicament is applied to the organism; passive cancer immune therapy (by administering the passive immune-therapeutic drug, the TAA-specific MAB, to a patient in an effective amount) means that the effector mechanisms are already present in the cancer immune therapy. Effector mechanisms are the executing components of the immune system, mainly CTL and antibodies. Thus, the active cancer immune therapy (or the active component, active cancer immune medicament) primes TAA-specific CTL in vivo, whereas the passive cancer immune therapy (or the passive component, passive immune-therapeutic drug) is represented by a TAA-specific MAB manufactured ex vivo.

A TAA is for example HER2/neu which is expressed in about 20% of all breast cancers; EGFR in 80% of colon cancers; VEGF in metastatic or colorectal cancer; CAMPATH-1 and CD22 in B cell leukaemias; CA-125 in ovarian cancer; HLA-DR in acute lymphocytic leukaemia; chronic lymphocytic leukaemia, non-Hodgkin's lymphoma; Mucin-1 in colon cancer, lung cancer, breast cancer; carcino-embryonic antigen (CEA) in colon cancer, pancreatic cancer, breast cancer; survivin in many solid tumours; alpha-1-fetoprotein (AFP) in germ cell tumours; tyrosinase and melanoma-associated antigen (MAGE) in malignant melanoma.

Initially, TAAs were broadly classified into two categories based on their pattern of expression: tumour-specific antigens (TSAs), which are present only on tumour cells and not on any other cell; and TAAs, which are present on some tumour cells and also some normal cells. This classification, however, was imperfect because many antigens thought to be tumour-specific turned out to be expressed on some normal cells as well. The modern classification of tumour antigens is based on their molecular structure and source. According to the present invention, the term TAA is therefore encompassing both, classical TAAs and TSAs. Examples of TSAs, a sub-group of the TAAs according to the present invention, include the abnormal products of ras or p53 genes. Mutation of other genes unrelated to the tumour formation may lead to synthesis of abnormal proteins, which are also referred to as TAAs.

TAAs according to the present invention can generically be classified as products of mutated oncogenes and tumour suppressor genes, products of other mutated genes, over-expressed or aberrantly expressed cellular proteins, tumour antigens produced by oncogenic viruses, oncofetal proteins, altered cell surface glycolipids and glycoproteins, and/or cell type-specific differentiation antigens. Accordingly, any protein produced in a tumour cell that has an abnormal structure due to mutation can act as a TAA according to the present invention. Mutation of proto-oncogenes and tumour suppressors, which lead to production of abnormal protein, contribute to development of the tumour. Thus, such abnormal proteins are TAAs according to the present invention.

Proteins that are normally produced in very low quantities but whose production is dramatically increased in tumour cells, may trigger an immune response. An example of such a protein is the enzyme tyrosinase, which is required for melanin production. Normally tyrosinase is produced in minute quantities but its levels are very much elevated in melanoma cells. Oncofetal antigens are another important class of TAAs according to the present invention, e.g. AFP and CEA. These proteins are normally produced in the early stages of embryonic development and disappear by the time the immune system is fully developed. Thus self-tolerance does not develop against these antigens.

Cells infected with onco-viruses, e.g. EBV or HPV, also produce abnormal proteins. Cells infected by such onco-viruses contain viral DNA, which is transcribed and the resulting protein may trigger an immune response. In addition to proteins, other substances like cell surface glycolipids and glycoproteins may also have an abnormal structure in tumour cells and could thus become targets of the immune system and are therefore suitable as TAAs according to the present invention.

Against some TAAs, MAB medicaments have been developed; others are targeted by an active cancer immune medicament according to the present invention, e.g. for loading onto DCs or for direct application to a cancer patient together with an adjuvant. The TAA molecules targeted with MAB for the treatment of cancer according to the present invention may be on normal cells as well, but are e.g. over-expressed on tumour cells, which permit their use in tumour therapy. Because of over-expression of these TAAs, the MAB medicament accumulates in the tumour tissue where it may form immune complexes that cause activation of immune cells and the release of pro-inflammatory cytokines. The expression density of these tumour-associated molecules varies between patients. Consequently, also the amount of MAB molecules that are retained in the tumour tissue varies as it depends on the expression density of the tumour-associated antigen.

In principle, any form of active cancer immune medicament (for "active cancer immune therapy") may serve the purpose of initiating an anti-tumour immune response based on the immune system's cellular arm. The simplest form would be the use of a synthetic tumour antigen peptide or a recombinant protein together with an adjuvant. More complex are cancer vaccines based on whole wild type or genetically engineered autologous or allogeneic tumour cells. Protein, RNA, or DNA molecules extracted from autologous or allogeneic tumour cells may be used with or without an adjuvant. Synthetic or recombinant RNA and DNA molecules encoding tumour antigens may be used as active cancer immune medicament delivered via bacterial plasmids or recombinant viruses. Most preferred in the present invention as the active component of the TCIT is a DC-based active cancer immune medicament. The DCs of the TCIT are manufactured ex vivo and inoculated into a lymph node where they present tumour antigens to HTL and CTL. The primed T-cells leave the lymph node and search for the antigen they are responsive to. This search is facilitated by the pro-inflammatory cytokines released from the tumour tissue as a consequence of the MAB/TAA immune complex formation at the tumour site.

During the last years the DC has been recognised as the central regulator of immunity (Steinman & Banchereau (2007) Nature 449: 419-426). DCs are essential mediators between innate and adaptive immunity. They elicit immune responses upon invasion of pathogens. A switch of DC from their default tolerance maintaining to a pro-inflammatory and immune stimulatory mode of action, referred to as maturation, is initiated by pathogen-associated microbial pattern (PAMP) molecules (Medzhitov et al. (2002) Science 296, 298-300) binding to a molecule of the group of Toll-like receptors (TLR) such as lipopolysaccharide (LPS) to TLR-4 on the membrane of DCs (Dohnal et al. (2009) J Cell Mol Med 13: 125-135); damage-associated molecular patters (DAMP) (Schreiber et al. 2011, Science 331 1565-1570); pro-inflammatory cytokines (Jonuleit et al. (1997) Eur. J. Immunol. 27, 3135-3142), or CD40/CD40L signalling (Macagno et al. (2007) Trends Immunol. 28, 227-233).

In the early phases of a DC's maturation process initiated by the encounter of a TLR agonistic danger-signalling molecule, DCs acquire a pro-inflammatory mode of action, which is characterised by the secretion of TNF-α, IL-1 and - of critical importance - IL-12, molecules that contribute to the initiation and regulation of adaptive immune responses (Luger et al. (2013) PlosOne, 8: e54879). IL-12 is released for approximately one day after a DC's exposure to LPS (Langenkamp et al. (2000) Nat. Immunol. 1: 311-316). During that phase, IL-12 signalling to T cells polarises type 1 HTL-mediated immune responses that support CTL, the effector cells of the immune system's cellular arm (Felzmann et al. (2005) Cancer Immunol. Immunother. 54: 769-780; Hüttner et al. (2005) Cancer Immunol. Immunother. 54: 67-77).

PAMP recognition, must be stringently regulated, as excessive expression of signalling components as well as pro-inflammatory cytokines can have devastating effects on the host, resulting in chronic inflammatory diseases or autoimmune disorders. Most dramatically this is represented in case of bacterial sepsis, in which an overwhelming bacterial infection causes the massive release of LPS, a component of the bacterial cell wall. The family of LPS molecules is summarised as bacterial endotoxins. Binding of these LPS molecules to TLR-4 throughout the entire organism results in the massive release of pro-inflammatory cytokines including tumour necrosis factor alpha (TNF-α), frequently referred to as cytokine storm that may lethally damage inner organs.

In order to prevent an immune reaction from running out of control, approximately one day after encountering a PAMP danger-signalling molecule such as LPS, DCs assume an anti-inflammatory mode of action. This phase is characterised by enhanced activity of among others the tryptophan metabolising enzyme indoleamine 2,3 dioxygenase (IDO) and a high secretion level of IL-10, which supports an anti-inflammatory DC phenotype mediated by the autocrine IL-10/STAT3 signalling cascade. Both, IDO and IL-10 expressed in DCs contribute to the priming of immune suppressive regulatory T cells (Treg) (Jürgens et al. (2009) Blood 114: 3235-3243). The DC's anti-inflammatory mode of action terminates immune responses and acts as a safeguard preventing immune responses from running out of control. This suggests that the encounter between DCs and T-cells needs to take place within the one-day pro-inflammatory time window to allow efficient type 1 polarisation of HTL and activation of CTL (Felzmann et al. (2005) Cancer Immunol. Immunother. 54: 769-780; Hüttner et al. (2005) Cancer Immunol. Immunother. 54: 67-77). Interaction of DCs with T cells after their switch into an anti-inflammatory mode of action that takes place about 24 hours after encountering the PAMP danger-signalling molecule causes such T cells to assume an immune suppressive Treg phenotype. Consequently, the DCs of the active cancer immune medicament (the active component of the TCIT described here) need to be applied during the pro-inflammatory one-day time window, most preferred 6 hours after exposure to the PAMP danger-signalling molecule LPS (Dohnal et al. (2009) J. Cell. Mol. Med. 13: 125-135).

Per definition a mature DC is characterised phenotypically by maximum expression of - among others - the co-stimulatory CD80 and CD86 molecules, but doesn't take into account the secretion of IL-12. The expression of the co-stimulatory molecules reaches its maximum after 2 days. Thus, it takes the DCs 2 days to assume the defined status of full maturity, which, however, corresponds to the anti-inflammatory DC phenotype. Hence, it is important to distinguish between functionally different tolerance maintaining immature DCs, IL-12 secreting pro-inflammatory and immune stimulatory semi-mature DCs, and anti-inflammatory immune suppressive mature DCs (see e.g. WO2004024900A1, WO 2009/074341 A1). Encountering one of the maturation stimuli described above causes immature DCs to irreversibly commit to differentiate via the semi-mature DC phenotype lasting for one day into a mature DC phenotype within approximately 2 days.

A further aspect of the present invention relates to a method for producing DCs for the active cancer immune medicament component of the TCIT combined with a second passive immune-therapeutic drug component, a TAA-specific MAB, specifically for use as a TCIT combination treatment paradigm for cancer.

Optimised methods for generating DCs according to the present invention are disclosed in WO 2004/024900 A1, WO 2009/074341 A1, Felzmann et al. (2001) Cancer Letters 168: 145-154; Lehner et al. (2001) Blood 98: 736-742; Felzmann et al. (2002) Onkologie 25: 456-464; Felzmann et al. (2003) Cytotherapy 5: 391-398; Felzmann et al (2003) Hum Immunol 64: 762-770; Felzmann et al. (2005) Cancer Immunol Immunother 54: 769-780; Hüttner et al. (2005) Cancer Immunol Immunother 54: 67-77; Wimpissinger et al. (2006) J Clin Oncol 24: 14585; Dohnal et al. (2007) Cytotherapy 9: 755-770; Dohnal et al. (2009) J Cell Mol Med 13: 125-135; Dohnal et al. (2009) J Cell Mol Med 13: 1741-1750; Jurgens et al. (Blood) 114: 3235-3243; Traxlmayr et al. (2010) J Immunother 33: 40-52.

Differentiation from monocytes into immature DC, charging them with tumour antigen, and triggering maturation of the TAA-pulsed DCs may be done in subsequent steps of varying length and using various concentrations of the differentiation and maturation agents, or in combination of some or all steps of DC manufacturing. Such steps and variations are well available for a person skilled in the art and can be easily adapted for the specific TAA to be applied to the patient according to the present invention of a TCIT medicament (see also Jonuleit et al. (1997) Eur J Immunol 27: 3135-3142; Kalinski et al. (1999) Immunol Today 20: 561-567; Dohnal et al. (2009) J Cell Mol Med 13: 125-135; Boullart et al. (2008) Cancer Immunol Immunother 57: 1589-1597; Mills et al. (2009) BMC Cancer 9: 34; Bender et al. (1996) J Immunol Methods 196: 121-135; Thurner et al. (1999) J Immunol Methods 223: 1-15; Stift et al. (2003) J Clin Oncol 21: 135-142; Shankar et al. (2003) J Transl Med 1: 7; Rieser et al. (1997) J Exp Med 186: 1603-1608).

A preferred embodiment of the present invention is a combination wherein the active cancer immune medicament comprises a synthetic, recombinant, cell-derived autologous or allogeneic tumour protein, RNA or DNA molecules encoding one or more TAAs, preferably encoded and delivered via bacterial plasmids or viral vectors, the entire RNA extracted from tumour cells, autologous or allogeneic wild type or genetically engineered tumour cells, or combinations thereof. Most preferred as the active cancer immune medicament component in TCIT is a pharmaceutical preparation containing DCs that are exposed to any of the above listed material, have taken up this material, and processed it in order to deliver the information about the antigens to HTL and CTL.

Pharmaceutical DCs are prepared for administration to the patient. This pharmaceutical preparation of DCs fulfils all criteria necessary and currently described for applying DC containing preparations to a patient. DCs are manufactured ex vivo via differentiation from precursor cells such as haematopoietic, embryonic, or induced pluripotent stem cells. In a specifically preferred embodiment, the active cancer immune medicament in the combination according to the present invention comprises DCs that are differentiated from monocytes enriched from peripheral blood mononuclear cells (PBMC). Most preferred, PBMCs are collected via leukocyte apheresis, which is an extensively used clinical standard procedure, or are obtained by use of conventional blood collection. Monocyte enrichment from PBMCs is done using procedures well known in the art including plastic adherence, magnetic column selection of monocytes or depletion of non-monocytes in the PBMC preparation using MABs attached to magnetic beads, most preferred using counter flow centrifugation (elutriation). Monocytes are differentiated into DCs by cultivation in the presence of cytokines, preferably in the presence of type I/II interferons (IFN), interleukin (IL) - 13, IL-3, IL-4 and granulocyte macrophage-colony stimulating factor (GM-CSF), especially in the presence of a combination of IL-4 and GM-CSF. The differentiation of monocytes into DCs in the presence of a combination of IL-4 and GM-CSF is done for 2 to 14 days, preferably for 4 to 10 days, especially for 5 to 7 days. The DCs are prepared either from the cancer patient or from an allogeneic donor.

The DCs are exposed to TAAs, will phagocytose this protein, split it into short peptide fragments, load these fragments on MHC I/II molecules, and transport the MHC/peptide complexes back to the cell membrane where they are presented to T cells. In a preferred embodiment of the present invention, the active cancer immune medicament component of the TCIT is produced by loading synthetic, recombinant, or cell-derived autologous or allogeneic tumour protein, RNA, or DNA molecules encoding one or more TAAs or the entire RNA extracted from tumour cells, autologous or allogeneic wild type or genetically engineered tumour cells or tumour cell lysate, especially cells or cell lysate of a tumour patient who is supposed to be treated with the active cancer immune medicament, onto DCs. Loading of DCs is accomplished by relying on the phagocytic capacity of the DCs; or using chemical, physical or biological methods of transfection. After initiating the loading procedure, the DCs are incubated for at least 2 hours and up to 48 hours.

The DCs of the active cancer immune medicament component of the TCIT are preferably exposed to a maturation signal from the group of natural, synthetic, or recombinant PAMP danger-signalling molecules, which are TLR agonists including LPS, resiquimod (R848), imiquimod, poly(I:C), double-stranded RNA, CpG oligonucleotids, synthetic or natural prokaryotic DNA, flagellin, microorganisms dead or living, intact or fragmented (e.g. BCG), or combinations thereof; chemical, physical or biological stress or DAMP signals; cocktails of pro-inflammatory cytokines including but not limited to TNF-α, IL-1, IL-6, and the non-cytokine PG-E2; triggering of CD40-mediated maturation signals delivered via synthetic or recombinant CD40L molecules, cells or cell lines engineered to express CD40L molecules, T-cells or T-cell lines stimulated to express CD40L molecules, or stimulatory monoclonal antibodies directed at CD40. Maturation may be performed in the presence of type I/II IFNs, most preferred IFN-γ, IL-4, GM-CSF, but also other pro-inflammatory cytokines, or combinations thereof. Most preferred the DCs are contacted with LPS in the presence of IFN-γ that triggers maturation of the DCs characterised by IL-12 secretion for 24 hours. The maturation agent is applied after tumour antigen charging, preferably for up to 24 hours, more preferred for 2 to 12 hours, especially for 4 to 8 hours.

At the end of this production process, preferably after the incubation with the maturation agent, the DCs are frozen for storage and future administration. The DCs of the ready to use active cancer immune medicament component of the TCIT are divided into suitable aliquots, transferred into freezing containers, a commercial clinical grade freezing medium, e.g. a medium containing 2% DMSO, is preferably added, and the DCs can then be placed at minus 80°C. No special freezing procedure is necessary but can be used. Shelf life at -80°C is at least 2 years. Alternatively, DCs may be moved to the gaseous phase of a liquid nitrogen tank; the shelf life in a liquid nitrogen tank is also at least 2 years.

The active cancer immune medicament may be applied to a cancer patient in any suitable way known in the art. Preferred routes of administration are intravenously, intradermally, subcutaneously, intratumourally, into lymph nodes, or combinations thereof, preferably into lymph nodes. Preferably, the active cancer immune medicament is applied to a cancer patient together with an adjuvant, preferably an inorganic material, protein, RNA, DNA, microorganisms or eukaryotic autologous or allogeneic cells. Suitable adjuvants may generally improve the effectiveness of eliciting a TAA-specific immune response via the active component of the TCIT. The DC itself may be considered an adjuvant and is the most preferred option for the active component of the TCIT.

The TAAs in whatever form outlined above are not necessarily loaded on and presented from a DC. If the active cancer immune medicament is administered without DCs, it is preferred to bring it into the patient in such a way that the DCs of the patient take up the TAAs in vivo (i.e. the tumour protein, DNA, RNA, etc.). For example, the DCs residing in the skin of a patient can be addressed by subcutaneous or intradermal injection of the TAA material. The skin DCs will take up the antigen and proceed similarly as in the embodiment where DCs are loaded in vitro with the TAA. Adding LPS or a similar PAMP danger-signalling molecule to the TAAs will deliver the maturation inducing PAMP danger-signalling molecule to the skin DCs.

An active cancer immune medicament in combination with a passive MAB treatment effectively results in a targeted or guided cancer immune therapy according to the present invention. During the last decades, immunologic methods have become a central therapeutic strategy in the treatment of cancer. Probably the earliest example is the use of recombinant cytokines such as IL-2 in the treatment of melanoma (Lotze et al. (1986) Jama 256: 3117-3124). MABs were introduced into clinical applications in the late 1990s (Weiner et al. (2010) Nat Rev Immunol 10: 317-327). The first active vaccination approach was actually directed against a virus, the human papilloma virus (HPV), which is the cause of cervical cancer in almost all cases (Goldstone & Vuocolo (2012) Expert Rev Vaccines 11: 395-406). The first cancer immune therapy was approved in the year 2010 for the treatment of advanced prostate cancer: Sipuleucel-T (Provenge^{®}), which is an active cancer immune medicament based on antigen-presenting cells charged with a recombinant fusion protein comprised of the prostate cancer antigen prostate alkaline phosphatase (PAP) and GM-CSF (Kantoff et al. (2010) N Engl J Med 363: 411-422). In 2011, the MAB Ipilimumab (Yervoy^{®}) (Hodi et al. (2010) N Engl J Med 363: 711-723) with immune modulatory function was approved. It targets a molecule on the membrane of T cells that normally shuts them down, which is prohibited by Ipilimumab.

In case of a microbial infection, phagocytes, mainly DCs and macrophages, release pro-inflammatory cytokines at the infection site. The phagocytes become activated (i) by molecules representing a PAMP danger-signalling molecule that bind to receptors on phagocytes such as TLRs triggering the release of pro-inflammatory cytokines; or (ii) by immune complexes comprised of microbial antigens or intact microbes and specific antibody molecules together forming antigen/antibody immune complexes. Immune complexes bind to IgG Fc-receptors on phagocytes. The many IgG molecules that form the immune complex will crosslink the phagocytes' IgG Fc-receptors, which transmits an activating signal. Pro-inflammatory cytokines are released from the activated phagocytes that guide the antigen-specific T cells primed in lymph nodes towards the site of microbial invasion.

Unlike microorganisms, cancer tissue releases no danger-signalling molecules that could activate phagocytes. Also, under normal circumstances, there are no antibodies against tumour antigens and hence no immune complexes to activate phagocytes via binding to IgG Fc-receptors at the tumour site. DCs prime T cells in lymph nodes, which the T cells leave when activated in order to search and destroy their respective target. However, in the case of conventional active cancer immune therapy T cells have no guidance into the tumour tissue, as no cytokines are released from there, which may have a negative impact on the active cancer immune therapy.

In the TCIT paradigm this is complemented by the second component of the present invention, the TAA-specific MAB medicament. After application to the cancer patient, the MAB will accumulate at the tumour site forming immune complexes with TAAs. The tumour-resident phagocytes are induced to release pro-inflammatory cytokines via interaction of the MAB/TAA immune complexes with the IgG Fc-receptors. The pro-inflammatory cytokines guide TAA-specific T cells from the lymph node via the peripheral blood into the tumour tissue. The present invention, therefore, describes a TCIT that not only primes tumour antigen-specific T cells but also provides the means for these T cells to find their target thus potentiating the effectiveness of cancer immune therapy.

**Table 1: Examples of MABs for cancer treatment.**

| **Generic name** | **Trade name** | **Target** |
|---|---|---|
| **Un-conjugated antibodies** | | |
| Rituximab | Rituxan/Mabthera | CD20 |
| Trastuzumab | Herceptin | HER2 |
| Alemtuzumab | Campath/MabCampath; | CD52 |
| Cetuximab | Erbitux | EGFR |
| Bevacizumab | Avastin | VEGF |
| Panitumumab | Vectibix | EGFR |
| Ofatumumab | Arzerra | CD20 |
| ch14.18/CHO | | GD2 |

| **Immune-conjugates** | | |
|---|---|---|
| Gemtuzumab ozogamicin | Mylotarg | CD33 |
| 90Y-Ibritumomab tiuxetan | Zevalin | CD20 |
| Tositumomab and 131I-Tositumomab | Bexxar | CD20 |
| ch14.18-IL2 | | GD2 |

Most preferred in the present invention is the use of the MAB medicament Bevacizumab (Avastin^{®}) as the passive immune-therapeutic drug component of the TCIT paradigm according to the present invention. Theoretically, Bevacizumab may be used for the targeting of any type of cancer. It blocks vascular endothelial growth factor (VEGF) molecules that are released from tumour cells in order to stimulate blood vessel growth and vascularisation of the tumour tissue. It was found that the combination treatment of patients suffering from Glioblastoma multi-forme (GBM), an aggressive cancer of the central nervous system, with a DC-based active cancer immune medicament and Bevacizumab greatly improved overall survival of patients (for details see the Examples section). The MAB medicament Bevacizumab was applied according to the manufacturer's instructions. Also preferred is the MAB ch14.18/CHO and the immune-conjugate ch14.18-IL2 that is used in the treatment of Neuroblastoma, a cancerous disease of the peripheral nervous system.

The passive immune-therapeutic drug component utilises a TAA-specific MAB derived from the immune system's humoral arm for directing the T cells activated by the active cancer immune medicament into the tumour tissue. MAB are manufactured using standard technology by immunising animals with tumour antigens, identifying B-lymphocytes that produce a tumour antigen-specific MAB, obtain the DNA sequence of the IgG molecule's antigen-binding fragment (Fab), use recombinant DNA technology to humanise the animal's IgG molecule, and genetically engineer cell lines to secret high amounts of the therapeutic MAB. Alternatively to humanisation, a transgenic animal with a human IgG gene locus may be used.

In principle, any therapeutic MAB may be used as the TCIT's passive immune-therapeutic drug component of this invention. The MAB accumulates in the tumour tissue where it forms antigen/antibody complexes that cause inflammation via binding to IgG Fc-receptors on tumour-resident phagocytes thereby triggering the secretion of pro-inflammatory cytokines, which guide the activated T cells from the lymph nodes into the tumour tissue. In addition to Bevacizumab, specifically preferred MABs are directed against CD20 (e.g. Rituximab, Ofatumumab, 90Y-Ibritumomab tiuxetan, Tositumomab and 131I-Tositumomab), HER2 (e.g. Trastuzumab), CD52 (e.g. Alemtuzumab), EGFR (e.g. Cetuximab and Panitumumab), CD33 (e.g. Gemtuzumab ozogamicin), and the ganglioside GD2 (e.g. ch14.18, ch14.18-IL2). All these preferred MABs are specific for tumour cells by either binding to membrane molecules present on the tumour cell or to molecules released from the tumour cell.

As already stated above, a preferred embodiment of the combination according to the present invention comprises an anti-VEGF MAB directed against the tumour-associated antigen VEGF, especially Bevacizumab. Another preferred embodiment of the tumour-specific MAB is a ch14.18/CHO antibody, preferably the immune conjugate ch14.18-IL2.

The central indication of the combination according to the present invention is the use in the treatment of cancer. In general, the term "cancer" is used synonymously with "malignant disease", "malignant neoplasms" or "malignant tumours", which is also meant herein when referring to "neoplastic disease", "neoplasms" or "tumours". Malignancy is a feature of any indication for treatment within the central indication, cancer, of the present invention. It refers to any kind of malignant neoplastic disease that is characterised by uncontrolled proliferation of any type of cells and includes solid (malignant) tumour as well as leukaemias.

Preferably, the TCIT paradigm according to the present invention is used in the treatment of minimal residual malignant neoplastic disease, frequently referred to as minimal residual disease (MRD) without specifying that these are the residues of a malignant neoplasm. The term MRD is used in solid tumours as well as leukaemias that underwent treatment such as surgery, radiotherapy, and/or chemotherapy that succeeded in eliminating the majority of the tumour mass. The tumour cells remaining in the patients are no longer detectable using conventional macroscopic imaging or similar examinations. It is known from experience, however, that MRD may cause disease recurrence in selected cases. Since MRD may not be detected using conventional technologies, also patients with suspected MRD are treated with a cycle of adjuvant chemo- or radiotherapy that is supposed to eliminate MRD. This means that a considerable number of patients that actually do not have any MRD will be exposed to the adverse effects of usually aggressive adjuvant chemo- or radiotherapy. Using the TCIT treatment paradigm of the present invention has the capacity to eliminate or control the few remaining tumour cells of MRD without the side effects of chemo-or radiotherapy. Thus, a specific field of use of the present TCIT is the adjuvant treatment of patients with suspected MRD.

The term "adjuvant" as used in this context must not be confused with the term "adjuvant" in the context of a vaccine, where such adjuvant supports the immune response against an antigen in a vaccine. In the context of cancer treatment the term adjuvant refers to the supporting effect of a cycle of chemo-and/or radiotherapy that consolidates the surgical elimination of the tumour mass. Although in such cases the tumour is most likely completely removed, a cycle of chemo- and/or radiotherapy is applied in the adjuvant setting. Using the TCIT paradigm for the adjuvant consolidation treatment of MRD has the potential to spare patients from the toxic effects of chemotherapy and from radiation damage.

The TCIT combination medicament according to the present invention is specifically useful in the treatment of cancers of the central nervous system, especially Glioblastoma; or cancers of the peripheral nervous system, especially Neuroblastoma.

The TCIT combination medicament according to the present invention may be used together with other cancer treatment methods. The cancer patient who is treated with the TCIT medicament according to the present invention may additionally be treated with at least one other cancer treatment. It is also preferred to additionally treat the cancer patient with medicaments that aim at treating the side effects of conventional tumour therapy such as chemotherapy; e.g. medicaments containing Erythropoietin may be used for the treatment of anaemia; Granulocyte-Colony Stimulating Factor may be used for the treatment of leukopenia. Also, the use of pro-inflammatory cytokines, preferably cytokines that are directed at and needed by T cells, especially IL-2 and/or IFN-γ, may be used together with the TCIT medicament of the present invention. Moreover, it can be helpful to further treat the cancer patient with a medicament that prevents an immune response from being shut down by interfering with negative immune-regulatory feedback signalling. The first member of this group of medicaments is the MAB Ipilimumab (Yervoy^{®}).

In general, the active and the passive drug combination of the TCIT treatment paradigm according to the present invention are administered in an effective amount to a patient in need of such cancer treatment. Typical dosages and administration regimen are well available to a person skilled in the art and depend on the very nature of the active cancer immune medicament and the TAA-specific MAB for the passive immune-therapeutic drug. When using a DC-based active cancer immune medicament, the DCs are administered at least 3 times, more preferred at least 5 times, especially at least 10 times, in an amount of 1 x 10⁵ to 1 x 10⁷ cells, preferably of 5 x 10⁵ to 5 x 10⁶ cells, especially of 1 x 10⁶ to 5 x 10⁶ cells. The active cancer immune medicament, e.g. DCs but also other preparations of TAAs with or without an immune-enhancing adjuvant are preferably administered in weekly, bi-weekly or monthly intervals (or combinations thereof). The dosing scheme for each MAB differs. Hence the MAB is dosed according to the specifications of the MAB's manufacturer that is provided with the package containing the MAB medicament.

A specifically preferred embodiment of the present invention of the TCIT treatment paradigm relates to a combination of an active cancer immune medicament and a passive cancer immune-therapeutic drug for use in the treatment of a cancer patient, wherein
- the active cancer immune medicament contains DCs that are differentiated from monocytes in the presence of cytokines, especially in the presence of IL-4 and GM-GSF; loaded with a tumour antigen, especially with a lysate of tumour cells derived from the cancer patient to be treated; and matured by the incubation with a maturation agent, preferably a TLR agonist, especially LPS; wherein the maturation is preferably carried out in the presence of one or more pro-inflammatory cytokines, especially IFN-γ; and
- the passive immune-therapeutic drug is a MAB directed at a membrane TAA or an antigen released from the tumour cells into the tumour tissue, especially Bevacizumab.

The present invention also relates to a TCIT Kit comprised of a component for active immune stimulation, i.e. an active cancer immune medicament, e.g. a DC-based active cancer immune medicament, but any other means for triggering cellular anti-tumour immunity will be suitable as well; and a component for passive substitution of an immune effector mechanism, i.e. a passive immune-therapeutic drug, e.g. a MAB, but also adoptive immune therapy with ex vivo expanded tumour antigen-specific T cells may be used that home into the tumour tissue where they release pro-inflammatory cytokines. Application routes and schedules may be as described above; all components of a TCIT kit may be applied via the same or via different routes, at the same or at different time points.

The invention of the TCIT treatment paradigm according to the present invention is further described by the following examples and the figures, yet without being restricted thereto.

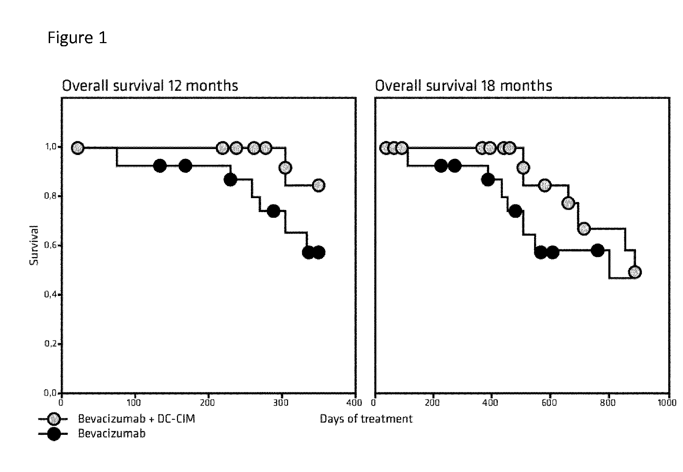

Figure 1 depicts the Kaplan-Maier curves for survival of patients at 12 (left) and at 18 (right) months; in black patients receiving standard treatment for GBM comprised of surgery, radiotherapy, and chemotherapy with Temozolomide that was switched to Bevacizumab upon disease relapse; in grey patients receiving standard treatment for GBM comprised of surgery, radiotherapy, and chemotherapy with Temozolomide that was switched to Bevacizumab upon disease relapse, and as add-on an active DC-based cancer immune medicament. It can be seen that addition of the DC-based cancer immune medicament considerably prolongs GBM patients' survival, especially at 12 months, but also at 18 months.

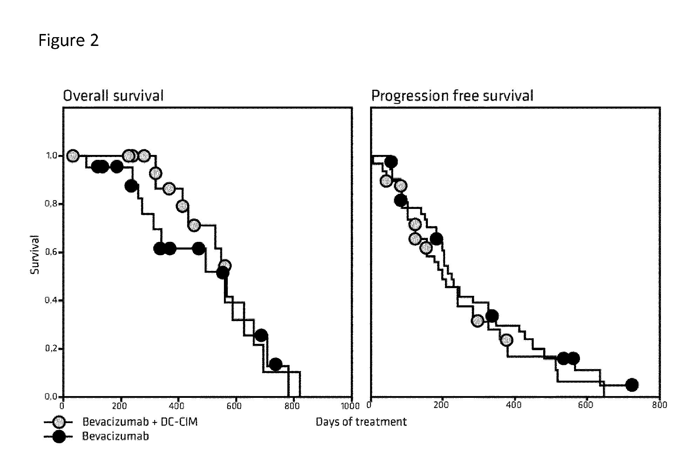

Figure 2 shows the Kaplan-Meier curves for overall (left) and progression free (right) survival; black and grey symbols as in figure 1. A clear trend is visible that addition of the DC-based cancer immune medicament prolongs GBM patients' overall survival; this trend is expected to become more robust as soon as more patients of the GBM-Vax study become evaluable. Progression free survival is not affected by the active DC-based cancer immune medicament.

### EXAMPLES

### CLINICAL TRIAL (GBM-VAX)

### Study design

The clinical trial that demonstrated the superior effectiveness of the TCIT combination medicament comprised of a DC-based cancer immune medicament (AV0113, described below) and a MAB (Bevacizumab) in the treatment of GBM was a randomised, open-label, two-arm, multi-centre, phase II clinical study (GBM-Vax). The treatment group received the standard therapy for GBM comprised of surgery for tumour mass reduction of at least 70%, radiotherapy, chemotherapy with Temozolomide switched to the MAB Bevacizumab as the passive immune-therapeutic drug component when the disease relapsed, and AV0113 as the active cancer immune medicament; the control group receiving standard therapy comprised of surgery for tumour mass reduction of at least 70%, chemotherapy with Temozolomide switched to Bevacizumab when the disease relapsed, and radiotherapy, but no AV0113.

### Rationale

The aim of the GBM-Vax study was to extend therapy options presently including surgery, irradiation, Temozolomide followed by Bevacizumab with an active DC-based cancer immune medicament in order to improve the poor prognosis of patients with GBM. In GBM patients 18-70 years it was aimed at delivering efficacy data for the efficacy of the AV0113 DC cancer immune medicament, which made randomisation into control and treatment groups necessary; in GBM patients 3-<18 and >70 years, safety and feasibility data were collected and hence the patients were not randomised.

### Objectives

- Overall survival measured as percentage of living patients with newly diagnosed GBM at 12, 18 and 24 months after a post-operative MRI scan treated according to the current standard (surgical resection, irradiation, oral chemotherapy with Temozolomide followed by intravenous therapy with Bevacizumab), and AV0113, an autologous DC-based cancer immune medicament, in which the DCs were charged with autologous tumour protein and matured with LPS and IFN-γ, as add-on therapy (group A), in comparison to patients receiving standard treatment and Bevacizumab without AV0113 (group B).
- Progression free survival measured as percentage of non-progressive patients with newly diagnosed GBM at 12, 18 and 24 months after a post-operative MRI scan receiving standard treatment and Bevacizumab together with AV0113 as add-on therapy (group A), in comparison to patients receiving standard treatment and Bevacizumab without AV0113 (group B).
- Quality of life in patients treated with standard therapy and Bevacizumab and AV0113 DC cancer immune therapy using ECOG (Eastern Cooperative Oncology Group) performance status compared to quality of life of patients receiving standard therapy and Bevacizumab but without AV0113 (for study patients older 18 years).
- Safety and feasibility of the combination of standard therapy, Bevacizumab and the AV0113 DC-based cancer immune medicament in GBM patients 3-<18 and >70 years of age.

### Number of subjects

A total of up to 76 male and female patients aged 18 to 70 years who meet all inclusion criteria and none of the exclusion criteria listed below were invited to participate in the study. The aim was to recruit 28 evaluable patients (no early progression) into each of the two treatment arms. Randomisation was based on stratification according to study sites at a 1:1 ratio.

Patients showing early disease progression confirmed by an MRI scan at 10 weeks or earlier after the post-operative MRI, were considered as "not evaluable" since progression occurred before the patient's immune system had sufficient time to respond to the first four priming immunisations with AV0113. Based on historical data from the Wagner-Jauregg Hospital, Linz, Austria, it was expected that up to 25% of the study subjects suffer early progression. In order to reach the number of 28 evaluable patients per treatment arm, it was expected to recruit up to 10 additional patients 18-70 years for both, control and treatment groups.

Patients 3 to 18 years were not randomised but received add-on therapy with AV0113. Paediatric patients didn't count towards the recruiting number of 2 x 28. Thus, the results obtained in paediatric GBM patients did not influence the outcome of the study in patients 18-70 years.

Additionally, safety and feasibility data for the AV0113 DC-based active cancer immune medicament in GBM patients >70 years of age were collected. Patients for this age group were included into the study until recruitment for the age group 18-70 years was completed. It was expected that up to 20 patients >70 years would be enrolled into the study.

### Inclusion criteria

- Female or male, paediatric or adult patients older than 3 years at time of diagnosis that qualify for GBM standard treatment.
- Primary GBM (WHO grade IV-VI), confirmed by pathohistology.
- Resection of at least 70% of tumour mass defined by post-operative brain MRI scan.
- Supra-tentorial tumour localisation.
- Life expectancy of at least 12 weeks by assessment of the attending physician.
- ECOG performance status 0, 1, or 2 (for study patients older 18 years).
- Written informed consent of patient and/or legal guardian in case of children and adolescents.

### Exclusion criteria

- Less than 100 µg of tumour protein obtained from the resected tissue for AV0113 production.
- Anti-neoplastic chemotherapy or radiotherapy during 4 weeks before entering the study, e.g. for the treatment of another neoplastic disease.
- Positive pregnancy test or breast-feeding.
- Patients unwilling to perform a save method of birth control.
- Known hypersensitivity to Temozolomide.

### Treatment arms

Patients 18-70 years were randomly assigned to 2 treatment arms of equal size:
A) Surgical tumour resection, standard radiotherapy, and Temozolomide switched to Bevacizumab upon disease recurrence, and with the AV0113 DC-based cancer immune medicament.
B) Surgical tumour resection, standard radiotherapy, Temozolomide with second line Bevacizumab therapy.

All patients 3-<18 years and >70 years received standard treatment and the AV0113 DC cancer immune medicament; these patients were not randomised and were analysed independently from patients 18-70 years for safety and feasibility.

### Manufacturing of AV0113

AV0113 is an active cancer immune medicament based on DCs. The details of DC manufacturing and quality control procedures are published (Dohnal et al. (2009) J. Cell. Mol. Med. 13: 125-135). Briefly, tumour tissue and peripheral blood leukocytes from the patient are required. Patients newly diagnosed with GBM undergo surgery for tumour mass reduction of at least 70%. Around 1 cm³ of the tumour tissue removed from the patient's brain is used. The tumour tissue is mechanically fragmented to obtain a single cell suspension of tumour cells. The number of cells obtained from this amount of tissue varies from patient to patient but is in all cases sufficient for the manufacturing of AV0113. In order to extract the proteins including the TAAs, the tumour cells are suspended in water and five times frozen and thawed. Particulate components remaining after the freeze/thaw procedure are pelleted and the cleared tumour protein/TAA solution is collected and frozen at -80°C.

PBMCs from the tumour patient are collected using leukocyte apheresis. This is a counter-flow centrifugation procedure ("blood centrifuge") that is used to separate the various components of blood, white blood cells, red blood cells, platelets, and serum. The patient is connected via an intravenous catheter to the machine. Blood flows continuously into the centrifugation chamber. The leukocyte apheresis machine is set up in a way that allows retention of white blood cells, PBMCs. All other components are returned to the patient via a second intravenous catheter. This permits the collection of large amounts of PBMCs, at least 10 billion. Monocytes are a subgroup of PBMCs of around 10%. At least 1 billion monocytes are needed for the manufacturing of one batch of AV0113.

Leukocyte apheresis is a clinical standard procedure, e.g. for the collection of human plasma. White blood cells are separated from peripheral blood in order to gather blood stem cells used in the transplantation for the treatment of leukaemia. The machine settings for the collection of monocytes are essentially identical to the settings used for the collection of monocytes. A physician specialised in blood transfusion medicine conducts the leukocyte apheresis procedure. Similar to tumour surgery, leukocyte apheresis is a clinical procedure that follows standard procedures well known in the medical profession.

The leukocyte apheresis product is delivered to the manufacturing facility. The majority of white blood cells in the leukocyte apheresis product are lymphocytes, T cells and B cells. Monocytes represent 10% of the leukocyte apheresis product and, hence, need to be enriched from the PBMCs to 80-90%. For that purpose, another counter-flow centrifugation procedure, elutriation, is used. In contrast to the leukocyte apheresis machine, the elutriation machine has the capacity to separate monocytes from lymphocytes and collect them in different fractions. The enriched monocytes are transferred into a cell culture system at a density of 1-2 million monocytes per 1 cm² of the culture flask and 1 ml of cell culture medium. A commercial clinical grade medium, CellGro, is used; no serum supplement is needed in the differentiation culture. The medium is supplemented with IL-4 [317 U/ml] and GM-CSF [1000 U/ml]. The culture is maintained for 5-7 days.

At the end of the differentiation culture the tumour protein/TAA solution is added to the DC culture at a protein concentration of 10 µg/ml and the culture is maintained for 2 hours. At this time, the DC culture is supplemented with LPS [200 U/ml] and INF-γ [50 ng/ml] and maintained for another 6 hours. Finally, the DCs are harvested from the cell culture, washed, counted, and divided into portions of 1-5 million DCs. Each DC portion is frozen in 100 µl of a commercial clinical grade freezing medium comprised of 2% dimethyl-sulfoxide in cell culture medium supplemented with 10% human serum albumin. The vials are stored at -80°C; no special freezing procedure is required.

For quality control of AV0113, one vial is recovered from freezing and subjected to sterility testing for bacteria, fungi, mycoplasma, and viruses according to the respective national legislation. Sterility testing is outsourced to medical diagnosis laboratories in order to obtain independent quality assurance. The potency of the DC preparation to stimulate T-cells is assessed using a co-cultivation of DCs from AV0113 with allogeneic PBMCs; as a readout procedure the proliferation of T-cells is measured according to a standard procedure, an "allogeneic mixed leukocyte reaction", well known in the art and published previously (Dohnal et al. (2009) J. Cell. Mol. Med. 13: 125-135). The maturation status of the DCs is determined using flow cytometric measurement of the expression density of the DC membrane molecules CD80, CD83, CD86, CD1a, CD14, MHC I, MHC II. Flow cytometry is a standard procedure in immunology and haematology and is widely used in the characterisation of white blood cells for medical diagnostic and experimental purposes; commercial standard kits were used for these tests. The last parameter that was determined for quality control is the secretion of IL-12 from the DCs in AV0113. This was determined using commercial standard ELISA kits.

### Application of AV0113

- Injection volumes were 100-500 µl; the application route was intranodal; each aliquot contained 1-5 x 10⁶ DCs.
- Active immune treatment was comprised of 4 AV0113 injections in weeks 7, 8, 9, and 10 after the initiation of 6 weeks of chemo-radiotherapy. Subsequently, patients received AV0113 in weeks 12, 16, 20, 24, 28, and 32 in parallel with the maintenance chemotherapy with Temozolomide and subsequently Bevacizumab.
- If the first 10 AV0113 injections were well tolerated and more than 10 aliquots of AV0113 were produced, patients could receive further AV0113 injections (as long as more medicament was available) at each patient evaluation visit (weeks 39, 52, 65, 78, 91, 104 ± 3 weeks), after they signed an addition to the Informed Consent Form (ICF), in which they specifically consented to these additional injections.
- The immunisation schedule continued unaltered upon disease recurrence, withdrawal of Temozolomide, and initiation of Bevacizumab therapy.
- Patients of both groups received supportive care for acute or chronic toxicity whenever indicated, including blood components or antibiotics, and other intervention as appropriate. All treatment administered concurrently were documented in the electronic Case Report Forms (eCRF). Particular attention was given to concurrent treatment that could influence the intended effects or mask side effects of the AV0113 ± Bevacizumab TCIT treatment.

### RESULTS

### Number of patients in the respective groups (Table 2)

At the current stage of the GBM-Vax study only part of the patients may be analysed. Overall survival may only be assessed for patients that have died. This information is given in the table below.

**Table 2: Number of patients in the GBM-Vax trial, status January 2013.**

| | **Total # per group** | **Total # deceased** | **# deceased 1 year** | **# deceased 1,5 years** |
|---|---|---|---|---|
| **AV0113 & Bevacizumab** | 19 | 11 | 2 | 6 |
| **AV0113** | 12 | 4 | 4 | 4 |
| **Control & Bevacizumab** | 19 | 11 | 6 | 7 |
| **Control** | 15 | 5 | 4 | 4 |
| **Total number** | 65 | 31 | 16 | 21 |

### Age by treatment group (Table 3)

In GBM, age is the strongest predictive biomarker. The older the patients are at diagnosis, the worse the clinical outcome. Younger patients live on average longer compared to elderly patients. The median age distribution in both treatment groups was well balanced.

**Table 3: Age of patients.**

| **Age (years)** | | **A: AV0113** | **B: Control** | **Total** |
|---|---|---|---|---|
| | **Mean** | 55,8 | 63,0 | 54,3 |
| | **SD** | 11,0 | 10,6 | 10,8 |
| | **Median** | 56, 5 | 53, 5 | 54,5 |
| | **Q1 / Q3** | 46.0 / 65.5 | 46.0 / 61,0 | 46.0/63.0 |
| | **Min / Max** | 33.0 / 75.0 | 21.0 / 70.0 | 21.0/75.0 |
| **Total** | | 32 | 34 | 66 |

### Percentage of tumour resection (Table 4)

The inclusion criteria stated that at least 70% of tumour mass reduction was required. Ideally, only minimal residual malignant neoplastic disease is present after surgery. However, this is possible only in a small percentage of cases. The prognosis of the patients is impacted by the amount of tumour tissue that is left behind after surgery. The table shows that both groups had a similar percentage of median tumour mass reduction.

**Table 4: Percentaae of tumour resection.**

| **Resection (%)** | | **A: AV0113** | **B: Control** | **Total** |
|---|---|---|---|---|
| | **Mean** | 92,8 | 88,9 | 90,8 |
| | **SD** | 6,9 | 9,0 | 8,2 |
| | **Median** | 92,5 | 90, | 90, |
| | **Q1 / Q3** | 90,0 / 100,0 | 85,0 / 95,0 | 90,0/ 99,0 |
| | **Min / Max** | 75,0 / 100,0 | 70,0 / 100,0 | 70,0 / 100,0 |
| **Total** | | 32 | 34 | 66 |

### Patient's gender (Table 5)

The patient's sex is similarly distributed in both groups. It is known that GBM more frequently affects men compared to women.

**Table 5: Gender.**

| **Sex** | | **A: AV0113** | **B: Control** | **Total** |
|---|---|---|---|---|
| | **Male** | 21 (65,6%) | 23 (67,6) | 44 (66,7) |
| | **Female** | 11 (34,4) | 11 (32,4) | 22 (33,3) |
| **Total** | | 32 | 34 | 66 |

### Overall survival

Overall survival was determined using Kaplan-Meier estimator statistics. Patient's survival was assessed in a comparison between the groups that received standard therapy and Bevacizumab with (grey) or without (black) AV0113. Each step in the curves represents one patient's death; the dots along the curves represent patients still alive ("at risk" of death) and therefore not yet included into the analysis of overall survival. As may be deduced from the number of dots along the curves, there are still many patients "at risk". The robustness of the data will improve with time.

Figure 1 depicts the survival of patients at 12 (left) and at 18 (right) months. Also in this analysis, the number of patients included is so far small. However, of particular interest is the improved survival of patients receiving AV0113 in addition to standard therapy and Bevacizumab compared to patients receiving only standard therapy and Bevacizumab (Table 6). This difference is borderline significant in the Kaplan-Maier estimator statistics (p = 0,0856). Once more patients of the GBM-Vax study may be analysed, this observation should be confirmed and a robust trend for the efficacy of AV0113 may be demonstrated. Survival at 18 months also revealed a clear trend towards improved survival of patients receiving AV0113 in addition to standard therapy and Bevacizumab compared to patients receiving only standard therapy and Bevacizumab. The difference was not statistically significant.

Figure 2 shows the Kaplan-Meier estimator curves for overall (left) and progression free (right) survival. Due to the small number of patients that may be included into the analysis at this time, none of these differences were significantly different in the Kaplan-Maier estimator statistics (Table 6). However, a clear trend emerged that addition of AV0113 to the standard therapy and Bevacizumab improved the patients' overall survival. No differences were found in progression free survival in any of the groups (Table 6).

**Table 6: Statistical significances in survival analvses.**

| **Overall survival** | AV0113 & Bevacizumab | AV0113 | Control & Bevacizumab |
|---|---|---|---|
| AV0113 | 0,5565 | | |
| Control & Bevacizumab | 0,4991 | 0,9286 | |
| Control | 0,6963 | 0,8112 | 0,5857 |

| **Overall survival 1 year** | | | |
|---|---|---|---|
| AV0113 | 0,0428 | | |
| Control & Bevacizumab | 0,0856 | 0,6174 | |
| Control | 0,1349 | 0,6677 | 0,9875 |

| **Overall survival 1,5 years** | | | |
|---|---|---|---|
| AV0113 | 0,1443 | | |
| Control & Bevacizumab | 0,4303 | 0,6950 | |
| Control | 0,8657 | 0,6677 | 0,7910 |

### Overall and progression free survival (Table 7)

Patients receiving the AV0113 cancer immune medicament in addition to standard therapy and Bevacizumab had a considerably longer overall survival compared to patients that received only standard therapy and Bevacizumab but not the cancer immune medicament AV0113; the difference was borderline significant (p=0.06), although only about one third of the patients could be analysed as the others had not yet progressed far enough through the study.

**Table 7: Mean overall and progression free survival in control and AV0113 treatment groups receiving standard therapy with or without Bevacizumab**

| **Overall survival** | **With Bevacizumab** | **All patients** | **Without Bevacizumab** |
|---|---|---|---|
| **AV0113 Mean ± SD (number of patients)** | 535 ± 155 (11) | 438 ± 205 (16) | 223 ± 113 (5) |
| **Control Mean ± SD (number of patients)** | 406 ± 224 (11) | 380 ± 210 (17) | 334 ± 193 (6) |
| **p-value** | 0,06 | 0,22 | 0,14 |

| **Progression free sur-vival** | **With Bevacizumab** | **All patients** | **Without Bevacizumab** |
|---|---|---|---|
| **AV0113 Mean ± SD (number of patients)** | 221 ± 165 (19) | 204 ± 155 (30) | 174 ± 138 (12) |
| **Control Mean ± SD (number of patients)** | 240 ± 139 (18) | 242 ± 158 (29) | 246 ± 192 (11) |
| **p-value** | 0,44 | 0,17 | 0,16 |

The present invention specifically discloses the following embodiments:
1. The combination of an active cancer immune medicament and a passive immune-therapeutic drug, wherein the passive immune-therapeutic drug is a monoclonal antibody (MAB) specific for a tumour-associated antigen (TAA).
2. The combination according to embodiment 1, wherein the active cancer immune medicament comprises a synthetic, recombinant, or cell-derived autologous or allogeneic tumour protein, RNA or DNA encoding one or more TAAs, one or more TAAs encoded by bacterial plasmids or recombinant viruses, or as autologous or allogeneic wild type or genetically engineered tumour cells, or combinations thereof.
3. The combination according to embodiment 1 or 2, wherein the active cancer immune medicament is applied to a cancer patient intravenously, intradermally, subcutaneously, intratumourally, into lymph nodes, or combinations thereof, preferably into lymph nodes.
4. The combination according to any one of embodiments 1 to 3, wherein the active cancer immune medicament is applied to a cancer patient together with an adjuvant, preferably an inorganic material, protein, RNA, DNA, a Toll-like receptor (TLR) agonist, living or dead, intact or fragmented bacteria, especially Bacillus Calmette-Guerin (BCG), or eukaryotic autologous or allogeneic wild type or genetically engineered cells.
5. The combination according to any one of embodiments 1 to 4, wherein the active cancer immune medicament comprises dendritic cells (DCs) and wherein the DCs are charged with tumour antigen, preferably with a synthetic, recombinant, or cell-derived autologous or allogeneic tumour protein, RNA or DNA encoding one or more TAAs, TAAs encoded by bacterial plasmids or recombinant viruses, or with autologous or allogeneic wild type or genetically engineered tumour cells or tumour cell lysate, especially cells or cell lysate of a tumour patient who is supposed to be treated with the active cancer immune medicament, via cultivation, or chemical, physical or biological methods of transfection, and incubated for at least 2 hours and up to 48 hours, wherein the DCs are prepared either from the cancer patient or from an allogeneic donor.
6. The combination according to any one of embodiments 1 to 5, wherein the active cancer immune medicament comprises DCs and wherein the DCs are differentiated from Peripheral Blood Mononuclear Cells (PBMCs), especially monocytes, by cultivation in the presence of cytokines, preferably in the presence of type I/II Interferons (IFN), Interleukin (IL) -13, IL-3, IL-4 and Granulocyte Macrophage-Colony Stimulating Factor (GM-CSF), especially in the presence of a combination of IL-4 and GM-CSF, for 2 to 14 days, preferably for 4 to 10 days, especially for 5 to 7 days.
7. The combination according to any one of embodiments 1 to 6, wherein the active cancer immune medicament comprises DCs and wherein the DCs are differentiated from stem or progenitor cells, especially blood stem cells, embryonic stem cells or induced pluripotent stem cells, by cultivation in the presence of cytokines, especially in the presence of a combination of IL-4 and GM-CSF, and/or stem cell factor, IL-3, IL-13, Transforming Growth Factor (TGF) -β, type I/II IFNs, and wherein the cultivation is conducted for 2 to 14 days, preferably for 4 to 10 days, especially 5 to 7 days.
8. The combination according to any one of embodiments 1 to 7, wherein the active cancer immune medicament comprises DCs and wherein the DCs are exposed to a maturation signal from the group of natural, synthetic, or recombinant TLR agonists such as Pathogen Associated Microbial Pattern (PAMP) danger-signalling molecules, living or dead, intact or fragmented bacteria, especially Bacillus Calmette-Guerin (BCG), chemical, physical or biological stress or danger-signalling molecules, cocktails of pro-inflammatory cytokines containing Tumour Necrosis Factor Alpha (TNF-α) and Prostaglandin E2, triggering of CD40-mediated maturation signals delivered via synthetic or recombinant CD40L molecules, cells engineered to express CD40L molecules, T-cells or T-cell lines stimulated to express CD40L molecules, or stimulatory MABs directed at CD40.
9. The combination according to embodiment 8, wherein the maturation signal is a natural or synthetic PAMP molecule that binds to a TLR.
10. The combination according to embodiment 8 or 9, wherein the maturation signal is lipopolysaccharide (LPS).
11. The combination according to any one of embodiments 1-10, wherein the maturation agent is applied after tumour antigen charging, preferably for up to 24 hours, more preferred for 2 to 12 hours, especially for 4 to 8 hours.
12. The combination according to any one of embodiments 8 to 11, wherein the maturation agent is applied together with one or more pro-inflammatory cytokines, especially IFN-γ.
13. The combination according to any one of embodiments 1 to 12, wherein the passive immune-therapeutic drug component is a TAA-specific MAB used for the treatment of cancer according to standard clinical practice, especially a MAB that specifically binds to membrane molecules of the tumour, or to molecules, that are released from the tumour into the tumour tissue.
14. The combination according to any one of embodiments 1 to 13, wherein the passive immune-therapeutic drug is a MAB blocking growth factors released into the tumour tissue, a MAB binding to growth factor receptors, or a MAB binding to a tumour-associated membrane molecule, especially VEGF, EGFR, HER2, CD20, CD52, CD33, HLA-DR, Mucin-1, or the ganglioside GD2.
15. The combination according to any one of embodiments 1 to 14, wherein the passive immune-therapeutic drug is Bevacizumab.
16. The combination according to any one of embodiments 1 to 15, wherein the passive immune-therapeutic drug is a ch14.18/CHO antibody, preferably the immune-conjugate ch14.18-IL2.
17. The combination according to any one of embodiments 1 to 16 for use in the treatment of cancer.
18. The combination according to any one of embodiments 1 to 17 for use in the treatment of minimal residual malignant neoplastic disease.
19. The combination according to any one of embodiments 1 to 18 for use in the adjuvant treatment of cancer.
20. The combination according to any one of embodiments 1 to 19 for use in the treatment of cancers of the central nervous system, especially Glioblastoma; or cancers of the peripheral nervous system, especially Neuroblastoma.
21. The combination according to any one of embodiments 1 to 20, wherein the cancer patient is additionally treated with at least one other cancer medicament.
22. The combination according to any one of embodiments 1 to 21, wherein the cancer patient is further treated with medicaments that aim at treating the side effects of conventional tumour therapy, especially side effects of chemotherapy, preferably medicaments containing Erythropoietin, Granulocyte-Colony Stimulating Factor, and/or pro-inflammatory cytokines, preferably cytokines that are directed at and needed by T cells, especially IL-2 and/or IFN-γ; preferably in combination with other components of the immune system, especially ex vivo expanded TAA-specific T cells.
23. The combination according to any one of embodiments 1 to 22, wherein the cancer patient is further treated with medicaments for preventing an immune response from being shut down by interfering with negative immune-regulatory feedback signalling, especially Ipilimumab.
24. The combination of an active cancer immune medicament and a passive immune-therapeutic drug for use in the treatment of a cancer patient, wherein
   - the active cancer immune medicament contains DCs that are differentiated from monocytes in the presence of cytokines, especially in the presence of IL-4 and GM-GSF; loaded with TAAs, especially with a lysate of tumour cells of the cancer patient to be treated; and matured by the incubation with a maturation agent, preferably a TLR agonist, especially LPS; wherein the maturation is preferably carried out in the presence of one or more pro-inflammatory cytokines, especially IFN-γ; and
   - the passive immune-therapeutic drug is a VEGF-specific MAB, especially Bevacizumab.

## Claims

1. The combination of an active cancer immune medicament and a passive immune-therapeutic drug, wherein the passive immune-therapeutic drug is a monoclonal antibody (MAB) specific for a tumour-associated antigen (TAA).

2. The combination according to claim 1, wherein the active cancer immune medicament comprises a synthetic, recombinant, or cell-derived autologous or allogeneic tumour protein, RNA/DNA encoding one or more TAAs, one or more TAAs encoded by bacterial plasmids or recombinant viruses, autologous or allogeneic wild type or genetically engineered tumour cells, or combinations thereof, in the presence or absence of an adjuvant for potentiating the TAA-specific immune response, preferably an inorganic material, protein, RNA, DNA, Toll-like receptor (TLR) agonist, living or dead, intact or fragmented bacteria, especially Bacillus Calmette-Guerin (BCG), or eukaryotic autologous or allogeneic wild type or genetically engineered cells.

3. The combination according to claims 1 or 2, wherein the active cancer immune medicament is applied to a cancer patient intravenously, intradermally, subcutaneously, intratumourally, into lymph nodes, or combinations thereof, preferably into lymph nodes.

4. The combination according to any one of claims 1 to 3, wherein the active cancer immune medicament comprises dendritic cells (DCs) and wherein the DCs are charged with TAA, preferably with a synthetic, recombinant, or cell-derived autologous or allogeneic tumour protein, RNA/DNA encoding one or more TAAs, one or more TAAs encoded by bacterial plasmids or recombinant viruses, autologous or allogeneic wild type or genetically engineered tumour cells, especially cells or cell lysate of a tumour patient who is supposed to be treated with the active cancer immune medicament, via cultivation, or chemical, physical or biological methods of transfection, and incubated for at least 2 hours and up to 48 hours, wherein the DCs are prepared either from the cancer patient or from an allogeneic donor.

5. The combination according to any one of claims 1 to 4, wherein the active cancer immune medicament comprises DCs and wherein the DCs are differentiated from monocytes enriched from Peripheral Blood Mononuclear Cells (PBMCs), by cultivation in the presence of cytokines, preferably in the presence of type 1 or 2 Interferons (IFN), Interleukin (IL) -13, IL-3, IL-4 and Granulocyte Macrophage-Colony Stimulating Factor (GM-CSF), especially in the presence of a combination of IL-4 and GM-CSF, for 2 to 14 days, preferably for 4 to 10 days, especially for 5 to 7 days.

6. The combination according to any one of claims 1 to 5, wherein the active cancer immune medicament comprises DCs and wherein the DCs are differentiated from stem or progenitor cells, especially blood stem cells, embryonic stem cells or induced pluripotent stem cells, by cultivation in the presence of cytokines, especially in the presence of a combination of IL-4 and GM-CSF, and/or stem cell factor, IL-3, IL-13, Transforming Growth Factor (TGF) -β, type 1 or 2 IFNs, and wherein the cultivation is conducted for 2 to 14 days, preferably for 4 to 10 days, especially 5 to 7 days.

7. The combination according to any one of claims 1 to 6, wherein the active cancer immune medicament comprises DCs and wherein the DCs are exposed to a maturation signal from the group of natural, synthetic, or recombinant TLR agonists, preferably Pathogen Associated Microbial Pattern (PAMP) danger-signalling molecules, living or dead, intact or fragmented bacteria, especially Bacillus Calmette-Guerin (BCG), chemical, physical or biological stress or danger-signalling molecules, cocktails of pro-inflammatory cytokines containing Tumour Necrosis Factor Alpha (TNF-α) and Prostaglandin E2, triggering of CD40-mediated maturation signals delivered via synthetic or recombinant CD40L molecules, cells engineered to express CD40L molecules, T-cells or T-cell lines stimulated to express CD40L molecules, or stimulatory MABs directed at CD40; preferably a natural or synthetic PAMP molecule, most preferred Lipopolysaccharide (LPS).

8. The combination according to claim 7, wherein the maturation agent is applied after TAA charging, preferably for up to 24 hours, more preferred for 2 to 12 hours, especially for 4 to 8 hours and wherein the maturation agent is applied together with one or more pro-inflammatory cytokines, especially IFN-γ.

9. The combination according to any one of claims 1 to 8, wherein the passive immune-therapeutic drug is a MAB blocking growth factors released into the tumour tissue, a MAB binding to growth factor receptors, or a MAB binding to a TAA membrane molecule, especially VEGF, EGFR, HER2, CD20, CD52, CD33, HLA-DR, Mucin-1, or the ganglioside GD2.

10. The combination according to any one of claims 1 to 9, wherein the passive immune-therapeutic drug is Bevacizumab or wherein the passive immune-therapeutic drug is a ch14.18/CHO antibody, preferably the immune-conjugate ch14.18-IL2.

11. The combination according to any one of claims 1 to 10 for use in the treatment of cancer, preferably in the treatment of cancers of the central nervous system, especially Glioblastoma; or cancers of the peripheral nervous system, especially Neuroblastoma.

12. The combination according to any one of claims 1 to 11 for use in the treatment of minimal residual malignant neoplastic disease.

13. The combination according to any one of claims 1 to 12 for use in the adjuvant treatment of advanced cancer.

14. The combination according to any one of claims 1 to 13 for use in the treatment of cancer with or without other interventions aimed at the treatment of cancer, preferably chemotherapy, radiotherapy, and surgery; with or without supportive therapy for the treatment of side effects, preferably anaemia (Erythropoietin) or leukopenia (Granulocyte-Colony Stimulating Factor); with or without pro-inflammatory cytokines for enhancing the immune response against the tumour antigens, preferably cytokines that are directed at and needed by T cells, especially IL-2 and/or IFN-γ; also in combination with other components of the immune system, preferably ex vivo expanded TAA-specific T cells; and with or without medicaments for preventing an immune response from being shut down by interfering with negative immune-regulatory feedback signalling, especially Ipilimumab.

15. The combination of an active cancer immune medicament and a passive immune-therapeutic drug for use in the treatment of a cancer patient, wherein
- the active cancer immune medicament contains DCs that are differentiated from monocytes in the presence of cytokines, especially in the presence of IL-4 and GM-GSF; loaded with a TAA, especially with a lysate of tumour cells of the cancer patient to be treated; and matured by the incubation with a maturation agent, preferably a TLR agonist, especially LPS; wherein the maturation is preferably carried out in the presence of one or more pro-inflammatory cytokines, especially IFN-γ; and
- the passive immune-therapeutic drug is a VEGF-specific MAB, especially Bevacizumab.
